# EUROPEAN PATENT APPLICATION

(11) **EP 4 413 986 A1**
(43) Date of publication of application: **14.08.2024**
(21) Application number: 24152337.2
(22) Date of filing: 17.01.2024
(51) Int. Cl.: A61K 31/5025, A61K 9/00, A61K 45/06, A61P 1/16, A61P 9/10, A61P 11/00, A61P 43/00

(54) **USE OF [1,2,4] TRIAZOLO [4,3-B] PYRIDAZINE DERIVATIVE FOR PREVENTING AND TREATING SENESCENCE AND SENESCENCE-RELATED DISEASES**

(30) Priority: 09.02.2023 CN 202310153067; 09.02.2023 CN 202310124966; 20.02.2023 CN 202310133147; 20.02.2023 CN 202310133146
(71) Applicant: The Eight Affiliated Hospital, Sun Yat-Sen University, Shenzhen City, Guangdong Province 518033 (CN)
(72) Inventor: XIE, Chen, Shenzhen City, Guangdong Province, 518033 (CN); CHEN, Yanlian, Shenzhen City, Guangdong Province, 518033 (CN); HUANG, Hui, Shenzhen City, Guangdong Province, 518033 (CN); ZHANG, Tingting, Shenzhen City, Guangdong Province, 518033 (CN)
(74) Representative: Hafner & Kohl PartmbB

(57) **Abstract**

The present disclosure relates to the field of medical technology, particularly relates to use of [1,2,4] triazolo [4,3-B] pyridazine derivatives for preventing and treating senescence and senescence-related diseases. In particular, the present disclosure finds that C1632 has significant anti-senescence, anti-fibrosis and anti-vascular calcification effects. C1632 is a known small molecule drug which can be dissolved in water, ethanol or DMSO, it has good thermal stability, low cytotoxicity, good safety profile, good pharmacokinetic properties, and can be flexibly administered in a variety of ways comprising oral administration, intraperitoneal injection, intramuscular injection and intravenous injection. C1632 can be used as a safe, effective and convenient candidate drug.

## Description

### TECHNICAL FIELD

The present disclosure relates to the field of medical technology, particularly relates to use of [1,2,4] triazolo [4,3-B] pyridazine derivative for preventing and treating senescence and senescence-related diseases.

### BACKGROUND

Cell aging is a timing-related cellular dysfunction. Senescent cells undergo degenerative changes in cellular structure, presenting with functional decline and hypometabolism. Although physiological senescence is a slow process, viral infection, radiation, various diseases and other factors can lead to pathological senescence and accelerate the body's aging.

In addition, during the decline in biological function of organism and the individual aging, various related diseases occur. For example, chronic brain degeneration can affect cognitive functions and increase the incidence of Alzheimer's disease and neurodegenerative diseases. Senescent cells usually highly express inflammatory factors and other molecules that regulate the immune response, and cause immune system disorders comprising immune decline and chronic inflammation, leading to autoimmune diseases, osteoarthritis, etc. Aging is accompanied by the injury of angiogenic function, and senescence reduces the antithrombotic function of endothelium; they are both the risk factors for cardiovascular diseases such as atherosclerosis and vascular calcification. The deterioration of islet cell function with aging will lead to the inability of the body to normally respond to blood glucose variation, resulting in a decline of the ability to regulate blood glucose (*i.e.,* glucose tolerance), causing glucose metabolism disorders and eventually developing into diabetes. In addition, cellular senescence is a stable cell cycle arrest; although senescent cells still have metabolic activity, they cannot proliferate, and they promote the excessive secretion of extracellular matrix proteins by secreting inflammatory factors and other methods, involving in the onset and progression of tissue and organ fibrosis.

Therefore, the development of effective senescent intervention strategies is of great significance to prevent and/or treat senescence and senescence-related diseases and reduce the morbidity.

### SUMMARY

The present disclosure aims to provide a new use of the small molecule compound [1,2,4] triazolo [4,3-B] pyridazine derivative (i.e., C1632), that is, for preventing and treating senescence and senescence-related diseases.

The inventors have previously disclosed the use of C1632 for treating the novel coronavirus (SARS-CoV-2)-related diseases and anti-cytokine storm. However, it should be understood that cytokine storm is a severe acute immune system disease which is usually caused by pathogen infection that stimulates the innate immune pathways of cells and prompts cells to express large amounts of inflammatory factors in a short period of time, and inflammatory factors have adverse effects such as changing body temperature, affecting systemic metabolism, increasing sputum and clogging the alveoli, ultimately leading to respiratory distress, multiple organ failures and even death in patients. The senescence and senescence-related diseases involved in the present disclosure are completely different from the cytokine storm and novel coronavirus infection involved in the prior art. In particular, the senescence-related diseases of the present disclosure refer to any functional changes in the organism or any tissues, organs or cells thereof that occur with a decline in reproductive function after the period of maximum reproductive function. This change is aging-related. Senescence-related diseases refer to any diseases, disorders, degenerations, tissue loss, or other unhealthy or abnormal conditions caused by or associated with senescence. Obviously, senescence-related diseases are significantly different from the acute and severe diseases mentioned in the prior art with exogenous substances as causative factors.

Specifically, the following technical solutions are provided in the present disclosure.

In a first aspect, the present disclosure provides use of [1,2,4] triazolo [4,3-B] pyridazine derivative in the preparation of a medicament for the prevention and/or treatment of senescence and/or senescence-related diseases, wherein the [1,2,4] triazolo [4,3-B] pyridazine derivative is the compound of formula (I) or a pharmaceutically acceptable salt thereof: 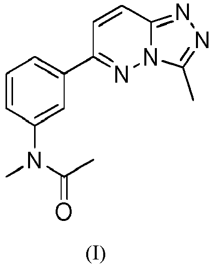 wherein the senescence-related diseases comprise at least one of atherosclerosis, vascular calcification, diabetes, autoimmune diseases, osteoarthritis, Alzheimer's disease, or tissue and organ fibrosis.

In a second aspect, the present disclosure provides a method of preventing and/or treating senescence and/or senescence-related diseases, comprising administering a therapeutically effective amount of [1,2,4] triazolo [4,3-B] pyridazine derivative to a subject in need thereof, wherein the [1,2,4] triazolo [4,3-B] pyridazine derivative is a compound of formula (I) or a pharmaceutically acceptable salt thereof: 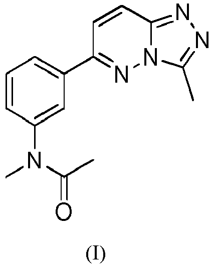 wherein the senescence-related diseases comprise at least one of atherosclerosis, vascular calcification, diabetes, autoimmune diseases, osteoarthritis, Alzheimer's disease, or tissue and organ fibrosis.

According to some embodiments of the disclosure, the senescence comprises at least one of senility, or senescence in tissues and organs comprising skin, brain, lung, heart, bone marrow, liver and kidney.

According to some embodiments of the disclosure, the [1,2,4] triazolo [4,3-B] pyridazine derivative inhibits cellular senescence.

According to some embodiments of the disclosure, the [1,2,4] triazolo [4,3-B] pyridazine derivative may extend lifespan.

According to some embodiments of the disclosure, the [1,2,4] triazolo [4,3-B] pyridazine derivative relieves an immunosuppressive microenvironment of senescent cells and/or promotes an immunologic clearance of senescent cells.

According to some embodiments of the disclosure, the vascular calcification is aortic vascular calcification.

According to some embodiments of the disclosure, the tissue and organ fibrosis comprises liver fibrosis, lung fibrosis, and kidney fibrosis.

According to some embodiments of the disclosure, the lung fibrosis is idiopathic pulmonary fibrosis.

According to some embodiments of the disclosure, the [1,2,4] triazolo [4,3-B] pyridazine derivative relieves alveolar reduction and reduces an elevated lung density during idiopathic pulmonary fibrosis..

According to some embodiments of the disclosure, the [1,2,4] triazolo [4,3-B] pyridazine derivative inhibits fibroblast proliferation.

According to some embodiments of the disclosure, the [1,2,4] triazolo [4,3-B] pyridazine derivative inhibits pulmonary hydroxyproline.

According to some embodiments of the disclosure, the [1,2,4] triazolo [4,3-B] pyridazine derivative inhibits immune cell infiltration. Preferably, the immune cells include CD45-positive cells (total immune cells), CD68-positive cells (macrophages), CD3-positive cells (T cells).

According to some embodiments of the disclosure, the kidney fibrosis is the kidney fibrosis caused by chronic kidney disease.

According to some embodiments of the disclosure, the [1,2,4] triazolo [4,3-B] pyridazine derivative inhibits tan abnormal expression of collagen fibers in chronic kidney disease.

According to some embodiments of the disclosure, the method of preventing and/or treating senescence and/or senescence-related diseases further comprises administering at least one of other components suitable for preventing and/or treating senescence and/or senescence-related diseases.

According to some embodiments of the disclosure, an administration route of the [1,2,4] triazolo [4,3-B] pyridazine derivative comprises at least one of intravenous injection, intraperitoneal injection, intramuscular injection, subcutaneous injection, oral administration, sublingual administration, nasal administration, nebulized administration, or transdermal administration.

In a third aspect, the present disclosure provides a method of preventing and/or treating senescence and/or senescence-related diseases, comprising administering a therapeutically effective amount of a medicament comprising [1,2,4] triazolo [4,3-B] pyridazine derivative to a subject in need thereof, wherein the [1,2,4] triazolo [4,3-B] pyridazine derivative is a compound of formula (I) or a pharmaceutically acceptable salt thereof: 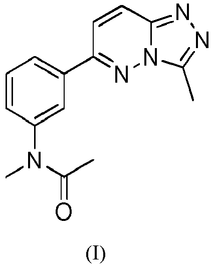 wherein the senescence-related diseases comprise at least one of atherosclerosis, vascular calcification, diabetes, autoimmune diseases, osteoarthritis, Alzheimer's disease, or tissue and organ fibrosis.

According to some embodiments of the disclosure, a dosage form of the medicament comprises at least one of suspension, granule, capsules powder, tablet, emulsion, solution, drop pill, injection, oral preparation, suppository, enema, aerosol, patch or drop.

According to some embodiments of the disclosure, an administration route of the medicament comprises at least one of intravenous injection, intraperitoneal injection, intramuscular injection, subcutaneous injection, oral administration, sublingual administration, nasal administration, nebulized administration, or transdermal administration.

According to some embodiments of the disclosure, the medicament further comprises pharmaceutically acceptable excipients.

Preferably, the pharmaceutically acceptable excipients comprise at least one of diluents, binders, wetting agents, lubricants, disintegrants, solvents, emulsifiers, co-solvents, solubilizing agents, preservatives, pH-adjusting agents, osmotic pressure regulators, surfactants, coating materials, antioxidants, bacteriostatic agents or buffers.

According to some embodiments of the invention, a dosage form of the medicament comprises at least one of suspension, granule, capsule, powder, tablet, emulsion, solution, drop pill, injection, oral preparation, suppository, enema, aerosol, patch or drop.

According to some embodiments of the invention, an administration route of the medicament comprises at least one of intravenous injection, intraperitoneal injection, intramuscular injection, subcutaneous injection, oral administration, sublingual administration, nasal administration, nebulized administration, or transdermal administration.

According to some embodiments of the disclosure, the medicament further comprises at least one of other components that may be used in the prevention and/or treatment of senescence and/or senescence-related diseases.

According to some embodiments of the disclosure, a dosage form of the medicament comprises at least one of suspension, granule, capsule, powder, tablet, emulsion, solution, drop pill, injection, oral preparations suppository, enema, aerosol, patch, or drop.

According to some embodiments of the disclosure, an administration route of the medicament comprises at least one of intravenous injection, intraperitoneal injection, intramuscular injection, subcutaneous injection, oral administration, sublingual administration, nasal administration, nebulized administration, or transdermal administration.

According to some embodiments of the disclosure, the medicament further comprises pharmaceutically acceptable excipients and at least one of other components that may be used in the prevention and/or treatment of senescence and/or senescence-related diseases.

According to some embodiments of the disclosure, a dosage form of the medicament comprises at least one of suspension, granule, capsule, powder, tablet, emulsion, solution, drop pill, injection, oral preparation, suppository, enema, aerosol, patch, or drop.

According to some embodiments of the disclosure, an administration route of the medicament comprises at least one of intravenous injection, intraperitoneal injection, intramuscular injection, subcutaneous injection, oral administration, sublingual administration, nasal administration, nebulized administration, or transdermal administration.

In a fourth aspect, the present disclosure provides a medicament comprising [1,2,4] triazolo [4,3-B] pyridazine derivative, wherein the [1,2,4] triazolo [4,3-B] pyridazine derivative is a compound of formula (I) or a pharmaceutically acceptable salt thereof; and the medicament is used for the prevention and/or treatment of senescence and/or senescence-related diseases:

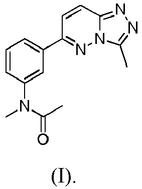

Preferably, the medicament further comprises at least one of other components that may be used in the prevention and/or treatment of senescence and/or senescence-related diseases.

Preferably, the medicament further comprises pharmaceutically acceptable excipients. More preferably, the pharmaceutically acceptable excipients comprises at least one of diluents, binders, wetting agents, lubricants, disintegrants, solvents, emulsifiers, co-solvents, solubilizing agents, preservatives, pH-adjusting agents, osmotic pressure regulators, surfactants, coating materials, antioxidants, bacteriostatic agents or buffers.

Preferably, a dosage form of the medicament comprises at least one of suspension, granule, capsule, powder, tablet, emulsion, solution, drop pill, injection, oral preparation, suppository, enema, aerosol, patch or drop.

Preferably, an administration route of the medicament comprises at least one of intravenous injection, intraperitoneal injection, intramuscular injection, subcutaneous injection, oral administration, sublingual administration, nasal administration, nebulized administration, or transdermal administration.

C1632 is a small molecule drug which can be dissolved in water, ethanol or DMSO, it has good thermal stability, low cytotoxicity, strong safety profile, good pharmacokinetic properties, and can be flexibly administered in a variety of ways.

The beneficial effects of the present disclosure are as below.

The inventors find that the [1,2,4] triazolo [4,3-B] pyridazine derivative (C1632) represented by formula (I) or a pharmaceutically acceptable salt thereof can prevent and/or treat senescence and/or senescence-related diseases. For example, C1632 can (1) significantly inhibit cellular senescence, extend lifespans of mice, inhibit lung senescence, relieve the immunosuppressive microenvironment during senescence, promotes immune clearance of senescent cells, and treats senescence-related diseases; (2) significantly inhibit the progression of liver fibrosis, have strong safety profile and anti-liver fibrosis effect, and can be used as a candidate drug for the treatment of liver fibrosis; (3) significantly inhibit the progression of idiopathic pulmonary fibrosis, reduce fibroblast proliferation and collagen synthesis during idiopathic pulmonary fibrosis, inhibit the activity of inflammatory infiltration of immune cells, protect alveolar architecture and reduce the elevated lung density during idiopathic pulmonary fibrosis, ameliorate lung density and ventilation; (4) significantly inhibit the progression of kidney fibrosis of mice caused by chronic kidney disease, ameliorate the weight loss of mice caused by chronic kidney disease and reduces the mortality of mice caused by chronic kidney disease; (5) ameliorate aortic vascular calcification and vascular compliance in mice.

In addition, C1632 is a known small molecule drug which can be dissolved in water, ethanol or DMSO, it has good thermal stability, low cytotoxicity, good safety profile, good pharmacokinetic properties, and can be flexibly administered in a variety of ways comprising oral administration, intraperitoneal injection, intramuscular injection and intravenous injection. C1632 can be used as a safe, effective and convenient candidate drug.

As used herein, the term "pharmaceutically acceptable" means that the compounds, materials, compositions and/or dosage forms are suitable for use in contact with human and animal tissues without excessive toxicity, irritation, allergic reaction or other problems or complications, and have a comparable and reasonable benefit/risk ratio, within the scope of sound medical judgment.

As used herein, the term "pharmaceutically acceptable salts" refers to salts formed by acidic functional groups present in the compound (e.g., -COOH, -OH, -SO₃H, etc.) with appropriate inorganic or organic cations (bases), including salts formed with alkali metal or alkaline earth metal, ammonium salts, and salts formed with nitrogenous organic bases; and salts formed by the basic functional groups present in the compound (e.g. -NH₂, etc.) with appropriate inorganic or organic anions (acids), including salts formed with inorganic or organic acids (e.g., carboxylic acids, etc.). These salts may be prepared during compound synthesis, isolation, purification, or alone using the free form of the purified compound in reaction with suitable acids or bases.

As used herein, the term "senescene-related diseases" refers to the degenerative or progressive lesions of tissues related to or caused by senescence, including tissues and organs fibrosis, neurodegenerative diseases, cardiovascular and cerebrovascular diseases, osteoarthritis, and chronic kidney diseases, etc. The aggregation of senescent cells is an important cause of senescence-related diseases. The abnormal aggregation of senescent cells leads to a decrease in the body's ability to maintain and restore homeostasis under stress and injury. Moreover, the inflammatory microenvironment created by senescence results in chronic tissue damage and accelerates senescence-related diseases such as atherosclerosis, vascular calcification, diabetes, autoimmune diseases, osteoarthritis, Alzheimer's disease and tissue and organ fibrosis. Alternatively, the term "senescene-related diseases" refers that the disease itself is not directly related to senescence, but is caused by external factors, senescence itself will change the prognosis of the disease such as viral infection, trauma, etc.

As used herein, the term "senility" refers to a congenital genetic disease which is mainly manifested as rapid acceleration of body aging, memory loss, and shortened life span.

As used herein, the term "tissue and organ fibrosis" refers to a progressive disease in which tissues and organs oversecrete extracellular matrix proteins to repair their damaged parts and form permanent scars, eventually leading to organ deformation and functional failure. It involves the formation of excess fibrous connective tissue in organs or tissues during repair or reaction. Although the fibrosis can be a benign state, the present disclosure preferably relates to a pathological state of fibrosis.

As used herein, the term "vascular calcification" (VC) refers to a disease characterized by the deposition of calcium salts in the intima and media of blood vessels. It is closely related to a variety of diseases such as atherosclerosis, diabetes and chronic kidney diseases, and seriously affects the prognosis of these diseases.

### BRIEF DESCRIPTION OF DRAWINGS

FIG.1A, FIG.1B and FIG.1C show that C1632 significantly inhibits cellular senescence in Example 1. FIG.1A shows β-gal staining, which demonstrates that C1632 treatment inhibits VP16-induced senescence in BJ cells and MRC5 cells. FIG. 1B shows the β-gal-positive cells rates of BJ cells in the different treatment groups. FIG. 1C shows the β-gal-positive cells rates of MRC5 cells in the different treatment groups. ** indicates p<0.01.
FIG.2A and FIG.2B shows that C1632 significantly extends the life span of mice and inhibits the lung senescence in senescent mice in Example 1. FIG.2A shows the survival curves, which shows that C1632 significantly extends the life span of progeria mice TERC-/- G4. FIG.2B shows that C1632 significantly inhibits the expression of lung senescence markers P16 and P21 in TERC-/- G4 mice as detected by immunohistochemistry.
FIG.3 shows the expression changes of the immunosuppressive T cell marker FOXP3 detected by immunohistochemistry in Example 1, demonstrating that C1632 can relieve the immunosuppressive microenvironment of senescence.

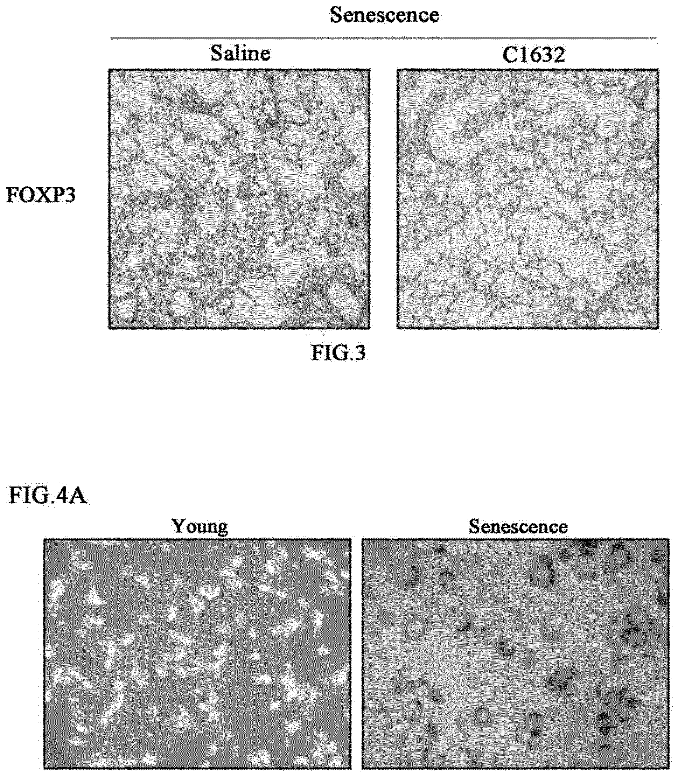

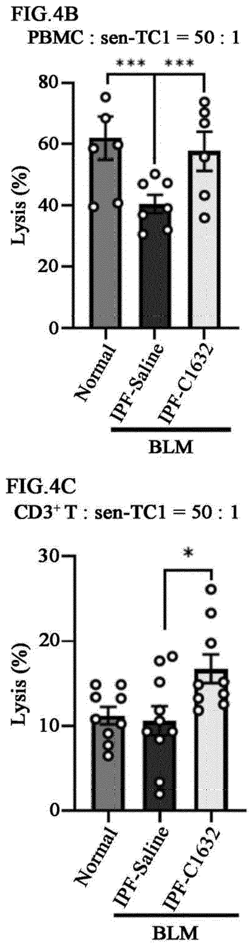

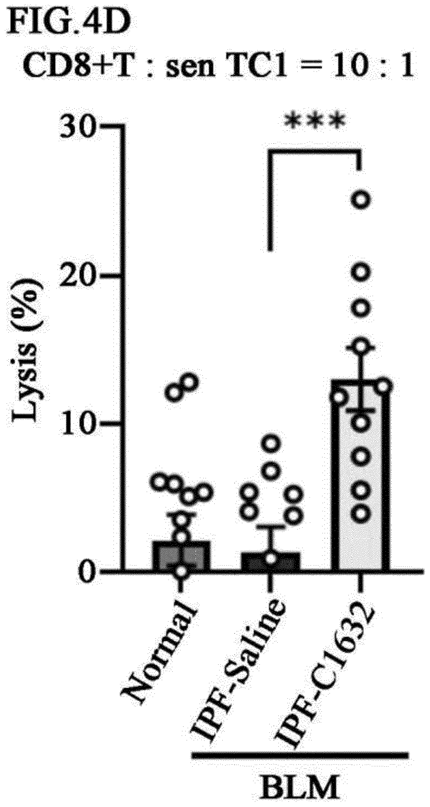

FIG.4A, FIG.4B, FIG.4C and FIG.4D show that C1632 promotes T cell to kill senescent cells in Example 1. FIG.4A shows the SA-β-gal detection of bleomycin-induced senescence in TC-1 cells. FIG.4B shows the killing effect of peripheral blood mononuclear cell (PBMC) on senescent TC-1 cells in normal mice, saline-treated idiopathic pulmonary fibrosis (IPF) mice, and C1632-treated IPF mice detected by LDH activity release assays; FIG.4C shows the killing effect of CD3+ T cells on senescent TC-1 cells in normal mice, saline-treated IPF mice, and C1632-treated IPF mice detected by LDH activity release assays; FIG.4D shows the killing effect of CD8+ T cells on senescent TC-1 cells in normal mice, saline-treated IPF mice, and C1632-treated IPF mice detected by LDH activity release assays.
FIG.5 shows the detection of collagen fibers in liver tissue through Masson staining in Example 2; 20× means 20 times magnification and 40× means 40 times magnification.
FIG.6 shows the detection of collagen fibers in liver tissue through VG staining in Example 2; 20× means 20 times magnification and 40× means 40 times magnification.
FIG.7 shows the detection of collagen fibers in liver tissue through reticular fiber staining in Example 2; 20× means 20 times magnification and 40× means 40 times magnification.
FIG.8 shows the detection of collagen fibers in liver tissue through Sirius red staining in Example 2; 20× means 20 times magnification and 40× means 40 times magnification.

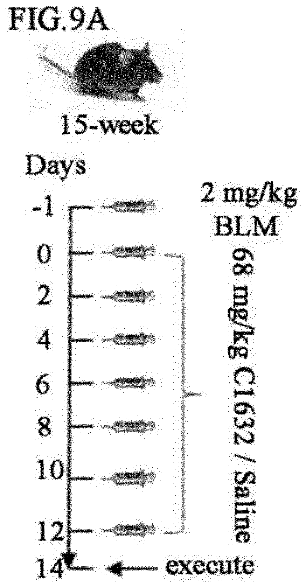

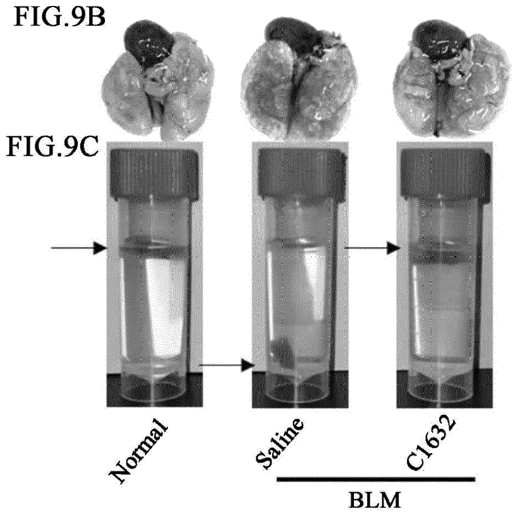

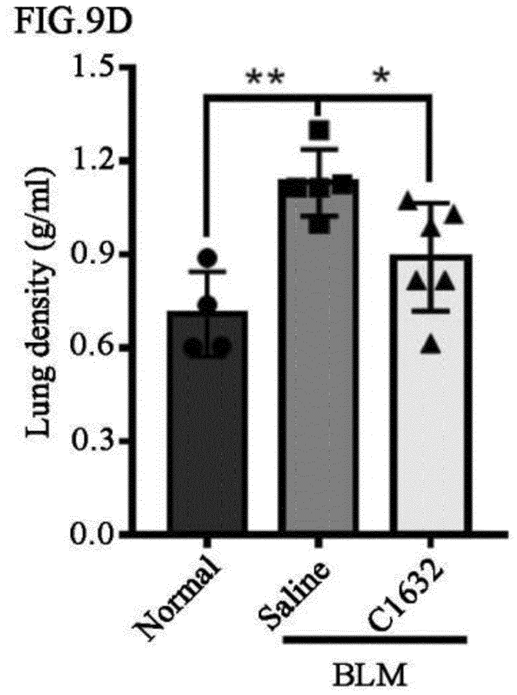

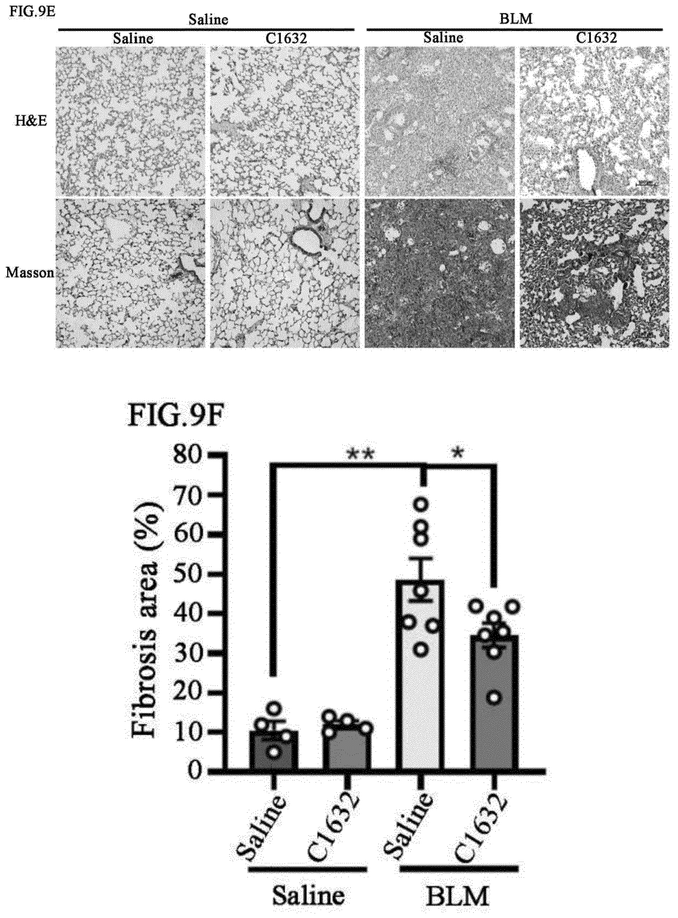

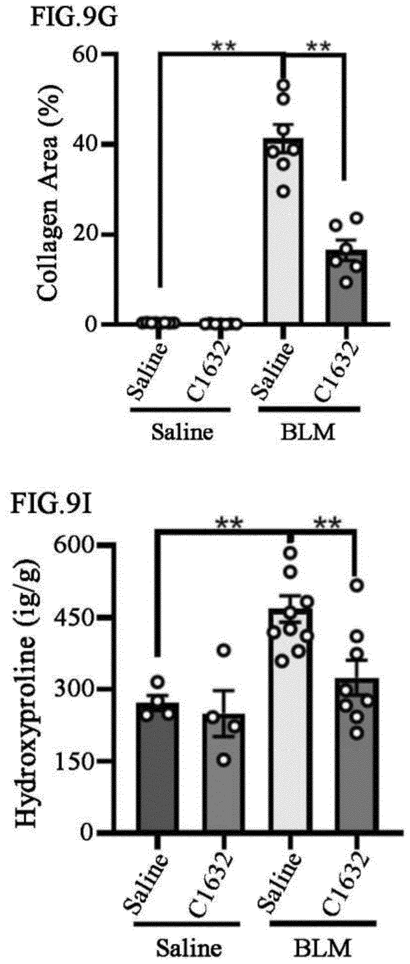

FIG.9A, FIG.9B, FIG.9C, FIG.9D, FIG.9E, FIG.9F, FIG.9G, and FIG.9I show that C1632 significantly inhibits the progression of pulmonary fibrosis in mice in Example 3. FIG.9A shows the flow chart of IPF mouse model construction and C1632 drug treatment experiment. FIG.9B shows photographs of lung tissues from normal mice, fibrotic mice, and fibrotic mice after C1632 treatment. FIG.9C shows that when the lungs of mice in each treatment group are placed in water, only the lung of IPF mice sinks since the lung density was higher than that of water, while the lung in the control group and C1632-treated group is less dense than water. FIG.9D shows the lung density statistics of mice in each treatment group. FIG.9E shows the mouse lung tissue architecture and fibrosis degree detected by HE and Masson staining. FIG.9F shows the fibrosis areas of mice in each group through HE staining. FIG.9G shows the fibrosis areas of mice in each group through Masson staining. FIG.9I shows the hydroxyproline content measured in mouse lung of each treatment group. Wherein, * indicates p<0.05 and ** indicates p<0.01.
FIG. 10A to FIG.10D show that C1632 inhibits immune infiltration in idiopathic pulmonary fibrosis in Example 3. FIG.10A shows the expression level of CD45, CD68 and CD3 in lung tissues of mice from the C1632-treated and control IPF groups detected by immunohistochemistry (IHC). FIG.10B shows the expression level of CD45. FIG.10C shows the expression level of CD68. FIG.10D shows the expression level of CD3.

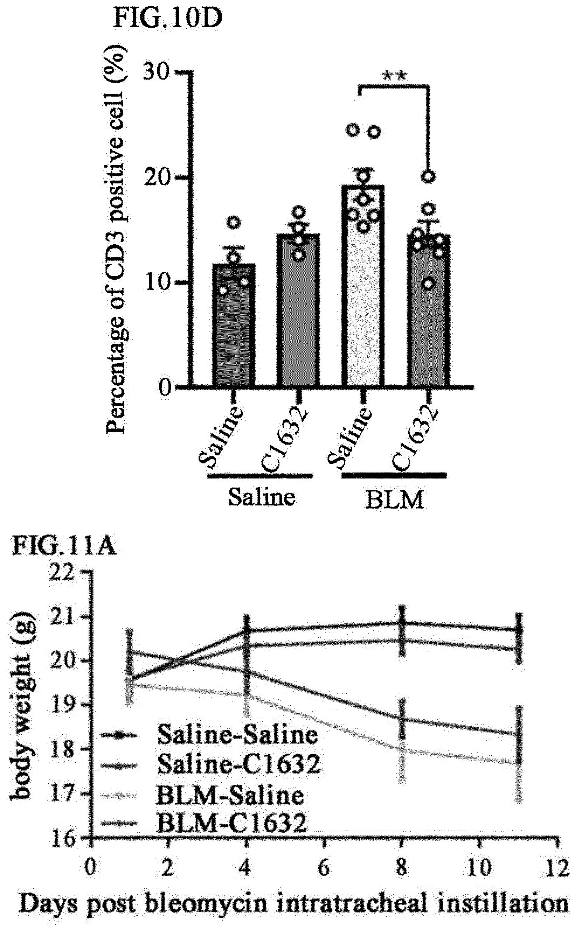

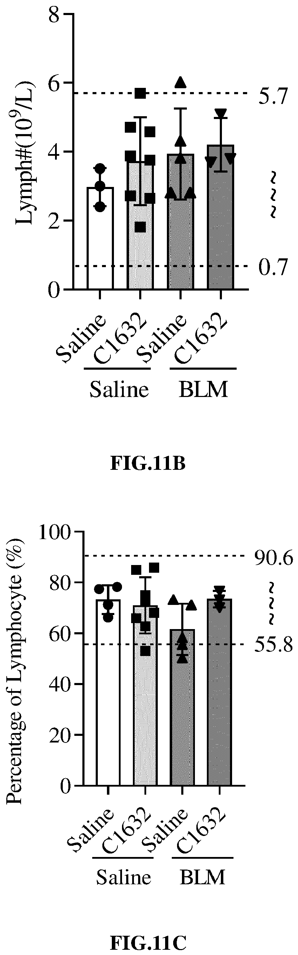

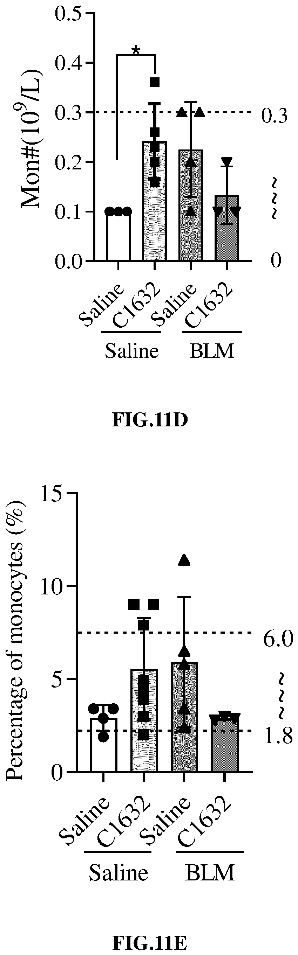

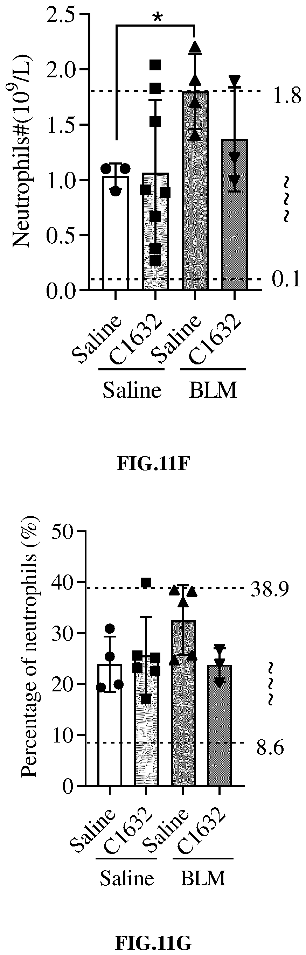

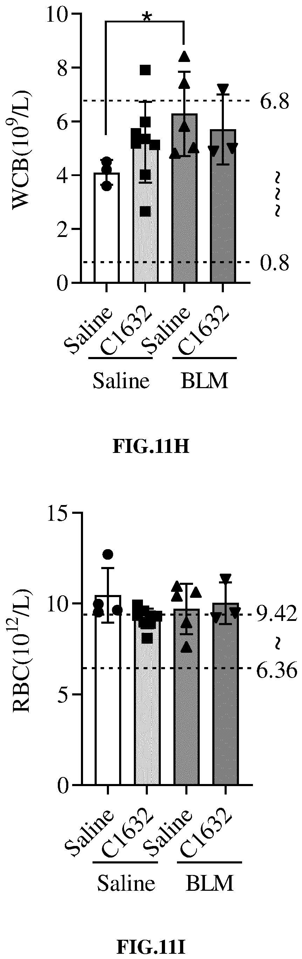

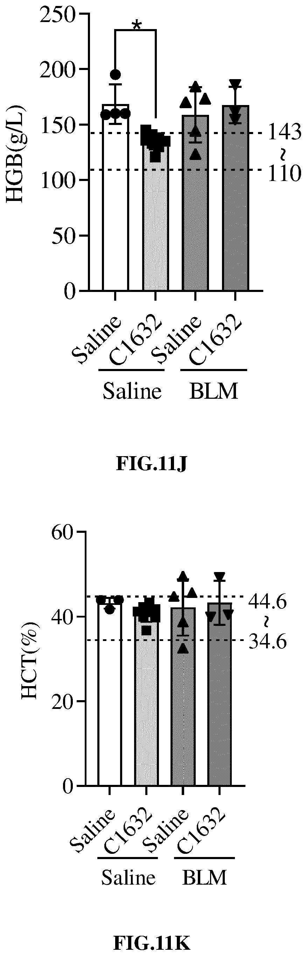

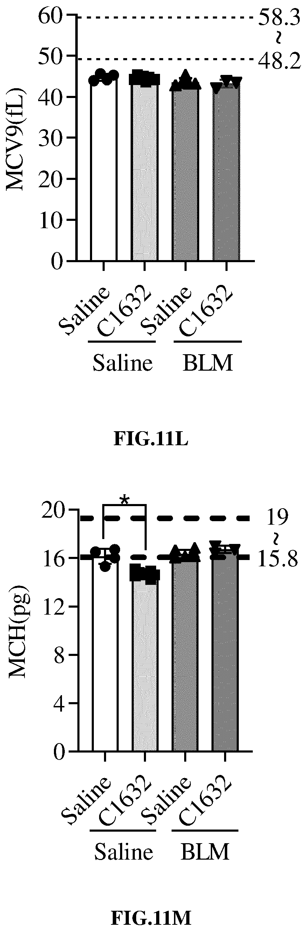

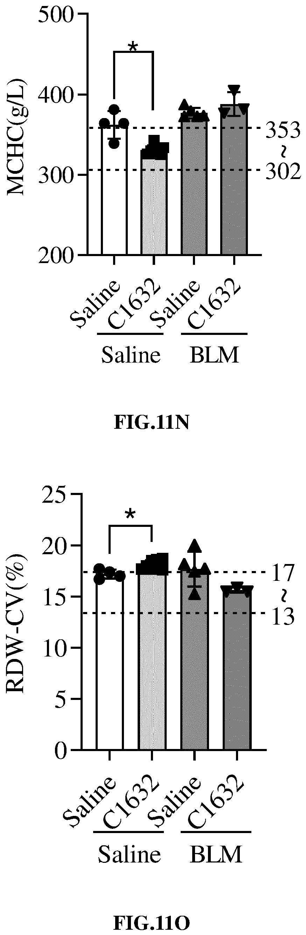

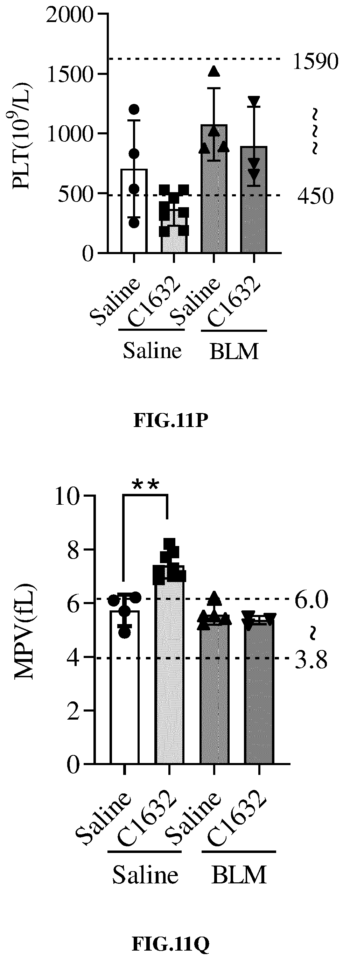

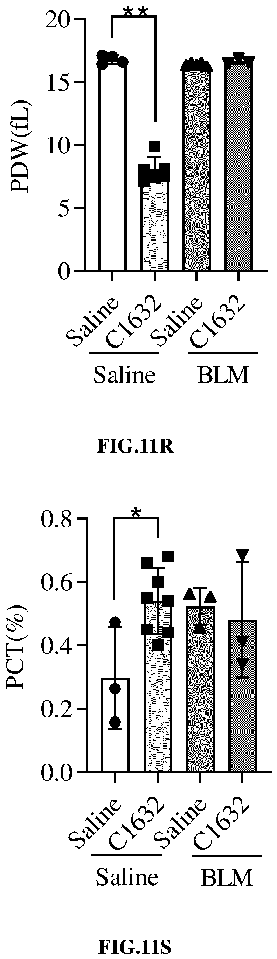

FIG.11A to FIG.11S show that C1632 has good safety profile in Example 3. FIG.11A shows the body weight statistics. FIG.11B to FIG.11S show routine blood analysis. FIG.11B shows lymphocyte density. FIG.11C shows the proportion of lymphocytes. FIG.11D shows the monocyte density. FIG.11E shows the proportion of monocytes. FIG.11F shows the neutrophil density. FIG.11G shows the proportion of neutrophils. FIG.11H shows the number of white blood cells. FIG.11I shows the number of red blood cells. FIG.11J shows the hemoglobin content. FIG.11K shows the hematocrit. FIG.11L shows the mean corpuscular volume. FIG.11M shows the mean corpuscular hemoglobin content. FIG.11N shows the mean corpuscular hemoglobin concentration. FIG.11O shows the red blood cell distribution width. FIG.11P shows the blood cell density. FIG.11Q shows the mean platelet volume. FIG.11R shows the width of platelet distribution. FIG.11S shows the platelet crit.
FIG.12 shows that C1632 treatment significantly inhibits the weight loss in mice caused by chronic kidney disease in Example 4. Wherein, ** indicates p<0.01.
FIG.13 shows that C1632 treatment significantly inhibits the death of mice caused by chronic kidney disease in Example 4. Wherein, ** indicates p<0.01.
FIG.14 shows C1632 significantly inhibits the progression of kidney fibrosis in mice with chronic kidney disease through Masson staining in Example 4. Wherein, ** indicates p<0.01.
FIG.15 shows that C1632 inhibits the progression of aorta vascular calcification in mice through vascular alizarin red staining in Example 5.

### DETAILED DESCRIPTION

The concept and resulting technical effects of the present disclosure will be clearly and completely described below with reference to the embodiments so as to fully understand the objects, features and effects of the present disclosure. Apparently, the described embodiments are merely a part rather than all of the embodiments of the present disclosure. All other embodiments obtained by a person of ordinary skill in the art based on the embodiments of the present disclosure without creative efforts shall fall within the scope of protection of the present disclosure.

Chemical synthesis of C1632: [1,2,4] triazolo [4,3-B] pyridazine derivative C1632 was synthesized in our laboratory and its structural formula is shown in formula (I) :

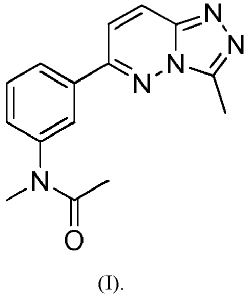

The synthetic route is shown below:

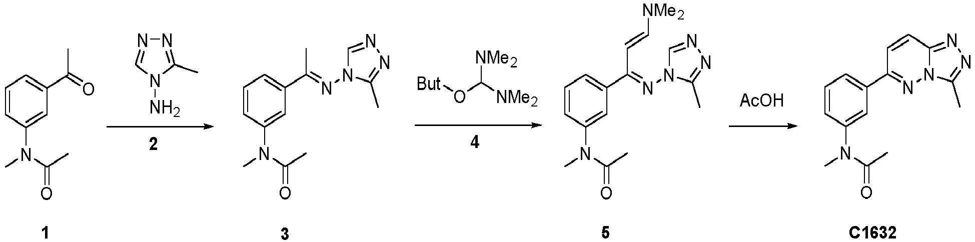

C1632 is a known small molecule drug which can be dissolved in water, ethanol or DMSO, it has good thermal stability, low cytotoxicity, good safety profile, good pharmacokinetic properties, and can be flexibly administered in a variety of ways comprising oral administration, intraperitoneal injection, intramuscular injection and intravenous injection.

### Example 1 Therapeutic effect of C1632 in senescence-related diseases

C57BL/6 mice used in this example were purchased from GemPharmatech. TERC-/- mice were provided by the laboratory of Professor Zhenyu Ju, Jinan University. Human primary cells MRC5 and BJ and mouse TC1 cells were purchased from ATCC.

The specific protocol for IHC testing in this example was as follows.

Paraffin sections were melted at 65°C for 1h, rehydrated by rinsing in xylene for 10min, xylene for 10min, absolute ethanol for 3min, 95% ethanol for 3min, 85% ethanol for 3min, 70% ethanol for 3min and water, followed by two rinses with PBS for 5 min each. The sections were blocked with 3% hydrogen peroxide (in methanol) for 10min, and then washed twice with PBS for 5min each time. Then the sections were put into antigen retrieval buffer (pH 6.0) and heat by microwave for 10min to retrieve antigen, then, slowly cool the solution at room temperature for 1h, and wash the solution twice with PBS for 5min each time. The sections were blocked with 10% goat serum for 1h, incubated overnight with the primary antibodies at 4°C in the dark, washed twice with PBS for 5min each time, incubated with the secondary antibodies at room temperature in the dark for 30min, and washed twice with PBS for 5min each time. The sections were subjected to DAB color development and hematoxylin staining, and then rinsed in tap water for 8min to produce a blue color. The sections were dehydrated with gradient alcohol, dried, and sealed with neutral gum.

1.1
MRC5 and BJ cells were cultured in DMEM low-glucose medium containing 10% FBS. The cells were passaged or plated at a ratio of 1:3. 0.5×10⁵ cells were plated in 12-well plates and in the next day treated with VP16 at a final concentration of 40 µM for 4-7 days to induce cellular senescence. A cellular senescence model was constructed. While inducing cellular senescence, C1632 with a final concentration of 120µM is added. The effect of C1632 on the senescence of BJ and MRCS cells was detected by SA-β-gal staining, and the effect of C1632 on cellular senescence was studied.

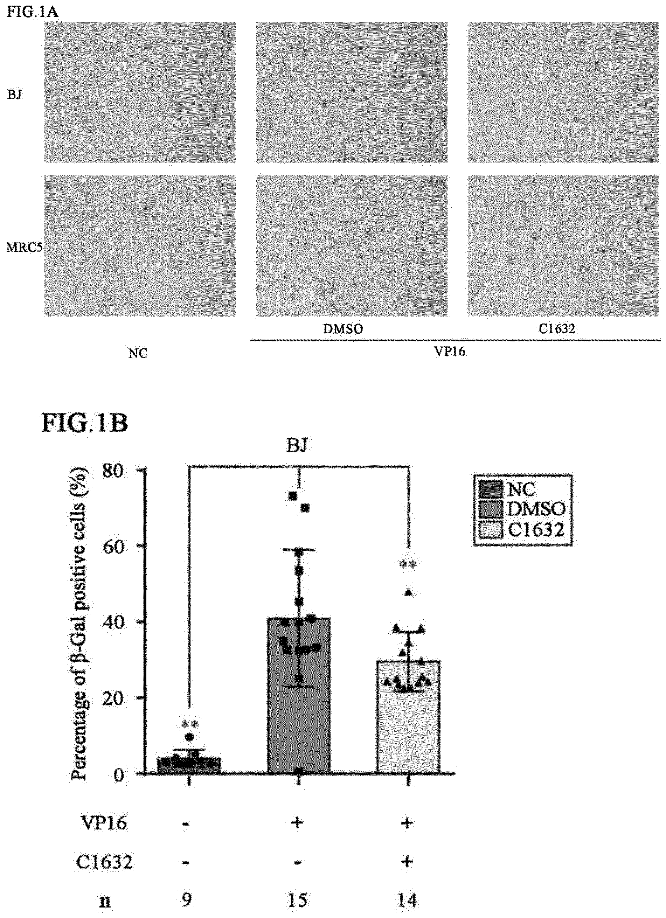

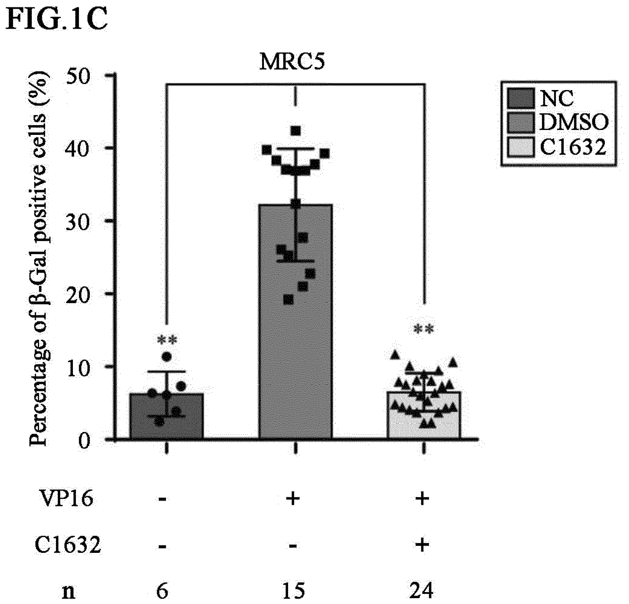

The results are shown in FIG. 1A, FIG. 1B and FIG.1C, wherein, FIG. 1A shows β-gal staining, which proves that C1632 treatment inhibits VP16-induced senescence in BJ cells and MRC5 cells. FIG.1B shows the β-gal-positive cell rates of BJ cells in the different treatment groups, and FIG.1C shows the β-gal-positive cell rates of MRC5 cells in the different treatment groups. ** indicates p<0.01. These results show that C1632 can effectively delay VP16-induced cellular senescence.

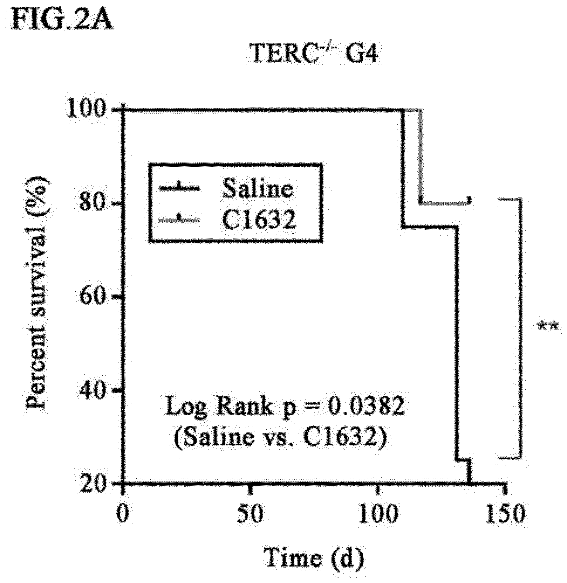

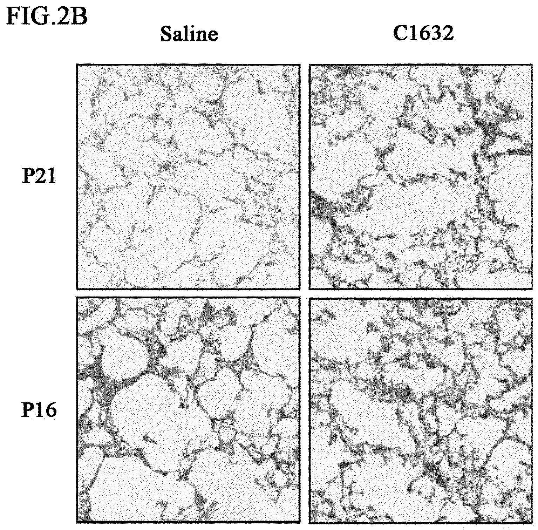

1.2
1. TERC-/ -G4 senescence mouse model: Mice had very long telomere length, and it took 3-6 generations of breeding to develop a premature senescence phenotype after telomerase knockout. TERC+/- mice were screened for TERC+/- mice as breeding protection. The first generation of TERC-/- mice was G1 mice. G1 mice were mated with each other to give birth to G2 mice, which continued to reproduce until G4. At this time, mice had very short telomeres and could develop senescence phenotype at 3-4 months of age.
2. Genotype identification of TERC knockout mice: Small segments of tails were cut from the identified mice and digested in 10mM Tris-HCl 0.5% SDS 1mM EDTA pH8.0 buffer containing 0.4mg/mL proteinase K at 65°C for 2 hours, and then 0.5µL digestive solution was used as a template for PCR amplification. PCR products which were 250bp indicated wild-type and 180 bp indicated TERC knockdown.
3. One-month-old G4 mice were given 68mg/kg C1632 once a week, with 10 mice in each group. All the mice in the control group died 3 and a half months after the experiment, while 80% of the C1632-treated mice still survived, indicating that C1632 greatly extended the life span of TERC-/ -G4 mice and reduced the risk of all-cause mortality (FIG.2A). Furthermore, the expression of senescence markers p16 and p21 was detected by immunohistochemistry in the lungs of mice, indicating that C1632 significantly inhibited lung tissue senescence (FIG.2B).
4. Since senescent cells were mainly eliminated by immune cells *in vivo,* in addition to inhibiting the generation of senescent cells, the inventors also examined the effect of C1632 on the immune microenvironment of senescent lung tissue. Immunohistochemical analysis of lung tissues from senescence mice shows that C1632 treatment reduces the immunosuppressive T cell marker FOXP3 in senescence tissues (FIG.3).

1.3
1. The senescence of TC-1 cells was induced with bleomycin, and the senescence of TC-1 cells was reflected through SA-β-Gal staining (FIG.4A).
2. cell killing assay: Bleomycin-induced senescent TC1 cells were plated at 10, 000/well in 96-well plates. The cell culture supernatant was discarded the next day, and then, PBMCS, CD3⁺ T cells, and CD8⁺ T cells isolated from normal mice, saline treated idiopathic pulmonary fibrosis (IPF) mice and C1632-treated IPF mice were add at a ratio of 1:50. After incubation at 37°C for 4h, the supernatantwas obtained after centrifugation. LDH enzyme was released from the killed senescent cells, and the killing effect of immune cells on senescent cells was detected by LDH kit. Bleomycin at a dose of 2 mg/kg was sprayed into the lungs of mice to construct IPF mouse model, each group of the above experiments had at least 6 mice.

Isolation and culture of CD8⁺T cells: Mouse spleens were ground under sterile conditions and filtered through a 200-mesh sieve. The filtered cells were sorted by CD8⁺ T cell isolation kit (biolegend). The sorted T cells were cultured in PRMI1640 medium containing 10%FBS, 5µg/mL anti-mouse CD28 antibody (biolegend), and 10 ng/mL IL-2 in the plates coated overnight with 5µg/mL CD3 antibody (biolegend) until use.

The results show that compared with saline-treated IPF mice, PBMC and CD3⁺ T cells from C1632-treated IPF mice have significantly enhanced killing activity against senescent TC-1 cells, in particular, the killing activity of CD8⁺ T cells from C1632-treated IPF mice against senescent cells is significantly higher than that from normal mice and saline-treated IPF mice (FIG.4B, FIG.4C, and FIG.4C). This suggests that C1632 significantly improves the targeted killing ability of the immune system against senescent cells and promotes the immune clearance of senescent cells.

### Example 2 Therapeutic effect of C1632 on liver fibrosis

C57BL/6 mice used in this example were 8 weeks old and purchased from GemPharmatech.

In order to prove whether C1632 can treat liver fibrosis, the mice were intraperitoneally injected with 1mL/kg carbon tetrachloride (CCl₄) twice a week for 6 consecutive weeks to establish a liver fibrosis model.

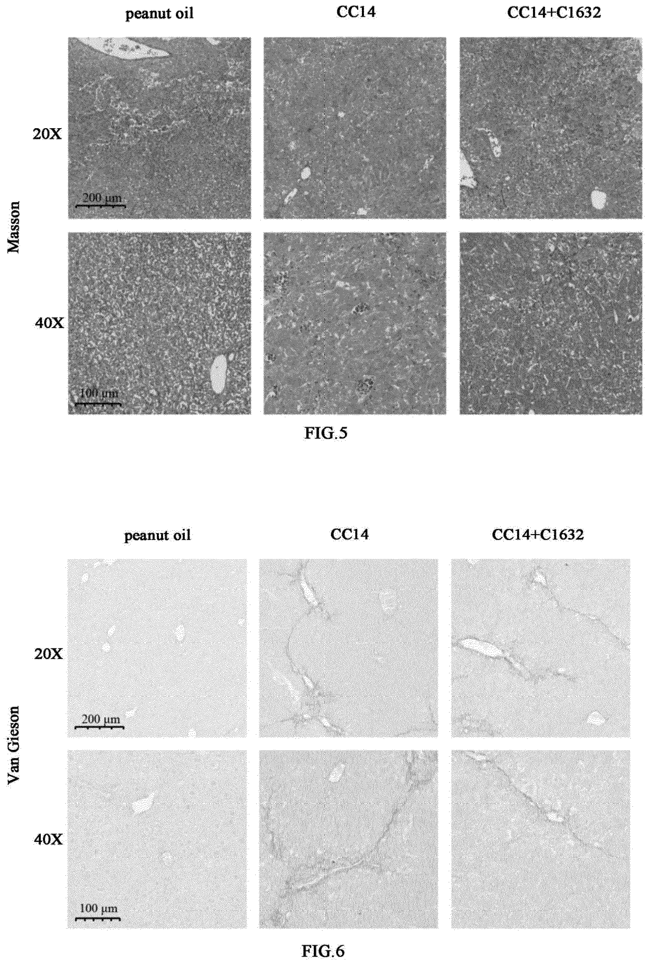

The specific steps were as follows:
C57BL/6 mice were divided into normal control group, CCl₄ group and CCl₄+C1632 group, with 7 mice in each group. The mice in the CCl₄+C1632 group were injected with 17 mg/kg C1632 for treatment after each injection of CCl₄. At the end of the experiment, the mice were sacrificed, and the obtained livers were fixed with 4% paraformaldehyde, dehydrated, embedded and sliced.
1. The collagen fibers in the tissue were stained blue through Masson staining, and the muscle fibers and cytoplasm were stained red through Masson staining. The sections were routinely deparaffinized to water and stained with Weigert's hematoxylin for 5 to 10 minutes. The sections were differentiated with 1% hydrochloric alcohol differentiation solution and washed with water. The sections was returned blue by using Masson bluing solution and washed with water for 1min. The sections were stained with Ponceau fuchsin staining solution for 5 to 10min, soaked in 1% glacial acetic acid for a moment, then washed with the phosphomolybdic acid solution for 1 to 2min. The sections were stained in the aniline blue staining solution for 1 to 2min after washed with 1% glacial acetic acid for 1min. The sections were rinsed with 1% glacial acetic acid in three consecutive cylinders for about 8 s, then successively dehydrated by three consecutive cylinders of absolute ethanol for about 5 s, 10 s, and 30 s, and dehydrated by two cylinders of n-butyl alcohol for 30 s and 2 min in turn, and finally permeabilizd by two cylinders of xylene for 5 min each time, and sealed with neutral gum. The results are shown in FIG.5. These results show that there are only a small amount of collagen fibers in the normal liver tissue, while a large amount of collagen fibers are expressed in the liver tissue of CCl₄-induced mice, and C1632 can inhibit the expression of collagen fibers induced by CCl₄ (FIG.5).
2. To further verify the effect of C1632 on liver fibrosis, the above tissue sections were subjected to Van Gieson (VG) staining, which was a mature connective tissue staining method to distinguish collagen fibers from muscle fibers. Paraffin sections were deparaffinized into water, and then placed into VG staining solution prepared with acid fuchsin and picric acid, stained for 1 min, quickly washed with water, and quickly dehydrated in three cylinders of absolute ethanol. The sections were placed into xylene for permeabilizing, then sealed with neutral gum and photographed under a microscope. The results show that CCl₄ increases the expression of collagen fibers in mice liver tissues, but C1632 significantly reduces collagen fiber levels in liver tissues (FIG.6).
3. Reticular fibers are less abundant in loose connective tissue, the fibers are fine, branched, and interwoven into a network. Reticular fibers have an equally spaced striated structure, and they are similar to collagen fibers with being composed of type III collagen. Reticular fibers are the main scaffolds for normal hepatocytes and hepatic sinusoids. The changes of reticular fibers can reflect the morphology of reticular scaffolds in liver lesions. According to the distribution characteristics and destruction of reticular fibers, the characteristics of regenerative nodules in cirrhotic liver, proliferative lesions, tumor and fibrosis degree can be displayed, and the progression of non-cirrhotic portal hypertension can also be periodically assessed. The collagen fibers of the reticular fibers are rich in glycoproteins and are argyrophilic which can be stained black.

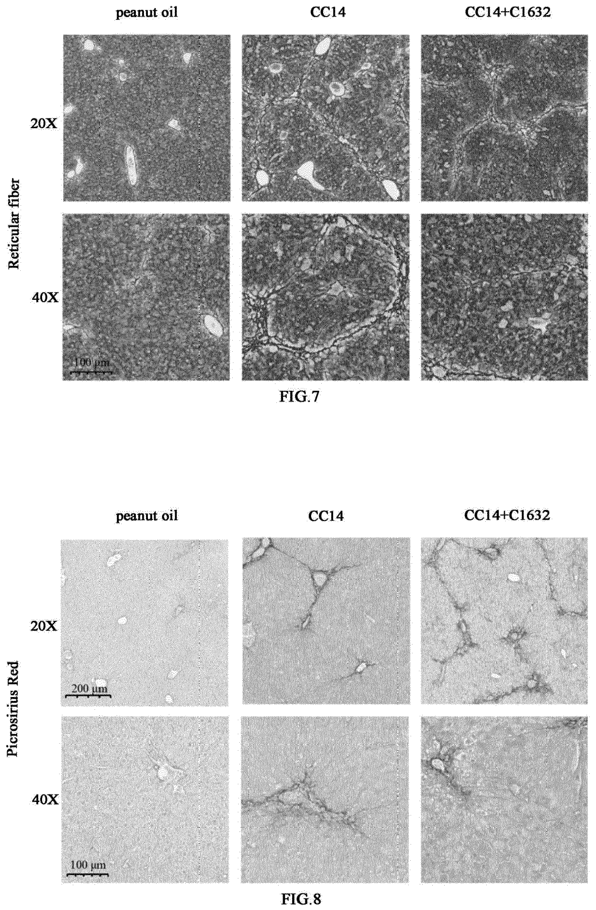

The sections deparaffinized to water were oxidized with Gordon-Sweets oxidizer for 5min. After washing with water, the sections were bleached with oxalic acid solution for 1 to 2min. After washing with distilled water, the sections were mordanted with ferric ammonium sulfate solution for 5min, then rinsed briefly with water. Gordon-Sweets silver ammonia solution was dropwise added for staining for 3min. The sections were briefly washed with distilled water, reduced with Gordon-Sweets reducing agent for 1min and rinsed under running water for 10min. The nucleus was stained with nuclear fast red staining solution for 5-10 min. After washing with water, the sections were dehydrated, permeabilized, and sealed with neutral gum. The results show that there are only a few reticular fibers in the normal liver tissue, while a large number of reticular fibers are expressed in the liver tissue of CCl₄-induced mice, and C1632 can inhibit the expression of reticular fibers induced by CCl₄ (FIG.7).

4. Picro sirius red staining is a simple and sensitive method for identifying the collagen fiber network in tissue sections, which qualitatively or quantitatively distinguish abnormal changes of collagen network in degenerative lesions and genetic or acquired diseases by means of morphological imaging analysis. Sirius red is a strong and linear anionic dye containing six sulfonic acid groups which are capable of firmly binding to cationic collagen fibers. Collagen fibers have a positive uniaxial birefringence property under polarized light detection. When combined with sirius red dye, the natural birefringence property of collagen is enhanced, allowing the distinction between type I collagen and type III collagen.

Paraffin sections were deparaffinized to water and stained with sirius red stain droplet for 1h. After washing with water, the nucleus was stained with Mayer's hematoxylin staining solution for 8 to 10min. After washing with water, the sections were routinely dehydrated and permeabilized, sealed with neutral resin. Under the polarized light microscope, type I collagen appeared strong orange yellow or bright red, and type III collagen appeared green. The results show that in the liver fibrosis model we constructed, CCl₄ mainly causes the production of type I collagen in the liver, and C1632 can significantly inhibit the production of type I collagen (FIG.8). In conclusion, C1632 treatment significantly reduces liver fibrosis and can effectively treat liver fibrosis.

### Example 3 Therapeutic effect of C1632 on kidney fibrosis

C57BL/6 mice used in this example were purchased from GemPharmatech. Bleomycin sulfate was purchased from Aladdin.

The method for constructing the IPF mouse model in this example was as follows.

A mouse model of kidney fibrosis was constructed by injecting bleomycin (2mg/kg) into the lungs of the mice through the airway after anesthesia.

The protocol for measuring mouse lung density in this example was as follows.

The lung density was measured by drainage method as follows:
① Sacrificing the mice after anesthesia, and weighing the lungs and recording as M_{lung}; ② weighing the empty 1.5mL centrifuge tube, filling with normal saline and the recording the weight of the centrifuge tube again; calculating the exact volume of the tube (Vtube) based on the weight and density of normal saline; ③completely immersing the lung tissue in the normal saline of the centrifuge tube, covering the lid of centrifuge tube after removing the air, wiping off the water stains around the tube, and recording the weight as M; ④ calculating the weight of normal saline in the tube: Mₛₐₗᵢₙₑ=M_{tube}-M_{lung}; ⑤calculating the volume of normal saline in the tube: Vₛₐₗᵢₙₑ=M_{saline×}ρₛₐₗᵢₙₑ, wherein, ρₛₐₗᵢₙₑ is the density of normal saline; ⑥calculating lung volume: V_{lung}=V_{tube}-Vₛₐₗᵢₙₑ; and ⑦calculating lung density: ρ_{lung}=M_{lung}/V_{lung}.

The protocol for immunohistochemical determination of immune cell infiltration in lung tissue in this example was as follows.

The lung tissues were fixed by immersion in 4% paraformaldehyde and then gradiently dehydrated in 70% ethanol for 5 hours, 80% ethanol for 1h, 90% ethanol for 1h, 95% ethanol for 1h, absolute ethanol for 1h, absolute ethanol for 0.5h, absolute ethanol +TO (1:1) for 1h, TO for 1h, TO for 1h, paraffin (62°C) for 1h, paraffin (62°C) for 1h, paraffin (62°C) for 1h. After dehydration, the lung tissues were wrapped in the wax blocks and sectioned with a thickness of 4µm. The paraffin sections were baked at 65°C for 1h and then exposed to xylene for 5min, xylene for 5min, absolute ethanol for 5min, 95% ethanol for 5min, 90% ethanol for 5min, 85% ethanol for 5min, 75% ethanol for 5min; then, gently boiled in citric acid solution for 10min and naturally cooled to room temperature, and exposed to 3% hydrogen peroxide for 10min. The sections were blocked with goat antiserum for 1h at room temperature, incubated overnight with primary antibodies against CD45 (abcam), CD3 (abcam) or CD68 (abcam), then washed 3 times with PBS for 5min each time, and then incubated with immunohistochemical secondary antibodies (Fujian Maixin) at room temperature for 20min. Then, the sections was subjected to color development for 2min by using DAB chromogenic kit (Fujian Maixin), rinsed with water to remove DAB, stained with hematoxylin for 2min and rinsed with running water for 8 minutes to return blue. After drying, the sections were sealed with neutral gum and photographed.

In this example, the degree of fibrosis in lung tissue was detected through Masson staining, using the Masson trichrome staining kit (Fujian Maixin).

3.1
To investigate whether C1632 can be used for the treatment of IPF, the inventors treated IPF mice with C1632 according to the method of FIG.9A, and divided the mice into ① normal mice + normal saline group; ② normal mice +C1632 group; ③IPF mice + normal saline group; and ④ IPF mice +C1632 group, each group had at least 4 mice. At the end of the experiment, the lung tissues of the mice were taken and photographed. The results show that the lungs of the control group are pink and smooth, without obvious abnormalities. The lungs of the model group are dark red, and some lung lobes are gray white, hardened, and scattered gray white plaques of different sizes could be seen. The lung shape, color and hardness of the model group treated with C1632 are closer to those of the control group (FIG.9B). The above-mentioned lung lobes were placed in water, and the results show that the lung of IPF mice is more dense than water, while the lung in the control group and C1632-treated group is less dense than water (FIG.9C).

Lung density of mice was further examined in each group, and the results show that IPF mice have greater lung density, which is significantly reduced by C1632 treatment (FIG.9D). These results suggest that C1632 can significantly improve the air content in the lungs of IPF mice, thereby reducing lung density and improving the ability of air exchange in the lungs. H&E and Masson staining also indicate that the lungs of C1632-treated mice contain more alveolar architecture and less fibrosis areas (FIG.9E to FIG.9G).

Hydroxyproline is a unique amino acid of collagen, and its content is an important indicator for detecting lung fibrosis. The quantitative results of hydroxyproline in the lung of mice in each treatment group show that C1632 could significantly inhibit the increase of hydroxyproline in the lung of mice (FIG.9I).

The results above indicate that C1632 significantly inhibits the progression of idiopathic pulmonary fibrosis in mice.

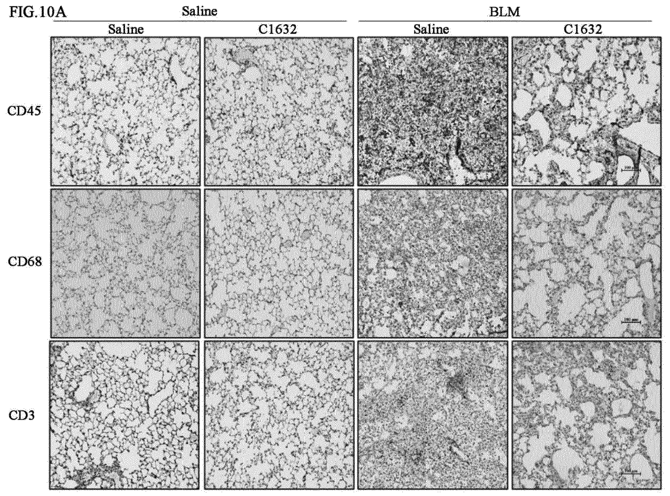

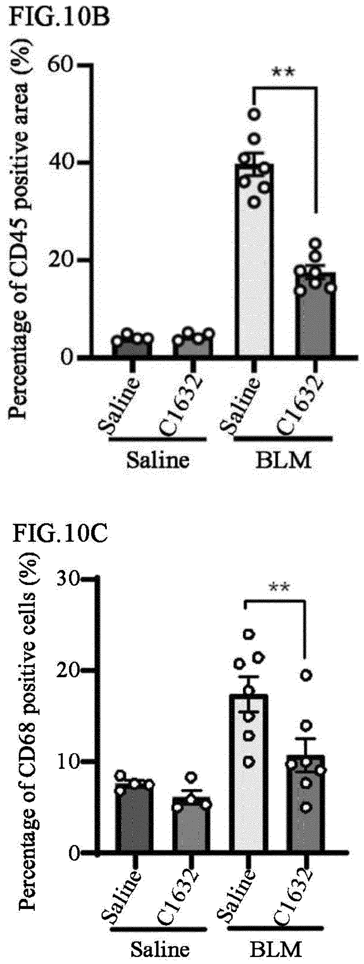

3.2
As illustrated in Example 3.1, C1632 may inhibit the progression of lung fibrosis, the inventors conjecture that it also inhibits the recruitment of immune cells during lung fibrosis. For this reason, the infiltration of CD45 (total immune cells), CD68 (macrophages) and CD3 (T cells) in the C1632-treated group were detected by IHC. The results show that injection of C1632 significantly reduces the infiltration of immune cells including macrophages and T cells in the lungs of IPF mice (FIG.10A to FIG.10D), indicating that C1632 inhibits immune infiltration in idiopathic pulmonary fibrosis.

3.3
In order to test the safety profile of C1632, the IPF model C57BL/6 mice and the control mice were intraperitoneally injected with 68mg/kg C1632 three times a week and weighed, respectively. At the end of the experiment, the whole blood wasa collected for routine blood test. The results show that C1632 does not significantly change the body weight of the mice compared with the corresponding saline group (FIG.11A). In addition, the routine blood test results of mice in each group are mostly within the reference interval. It is worth noting that C1632 administration to normal mice could significantly increase the mean platelet volume (MPV), but C1632 administration to model group has no significant effect on MPV However, C1632 significantly inhibits PDW (mean platelet volume) in normal mice, but has no significant effect on PDW in fibrotic mice. In general, C1632 does not cause deterioration in routine blood tests in IPF mice (FIG.11B to FIG.11S), indicating that C1632 has good safety profile.

### Example 4 Therapeutic effect of C1632 in kidney fibrosis

C57BL/6 mice used in this example were purchased from GemPharmatech. Feed containing 1.5‰ and 2‰ purine (AP) was processed by Guangdong Medical Laboratory Animal Center.

The protocol for constructing a chronic kidney disease (CKD) mouse model in this example was as follows: 8-week-old C57BL/6 male mice were fed with a feed containing 2‰ purine for 1 month, then, fed with a feedcontaining 1.5‰ purine for 3 months. There were at least 10 mice in each group.

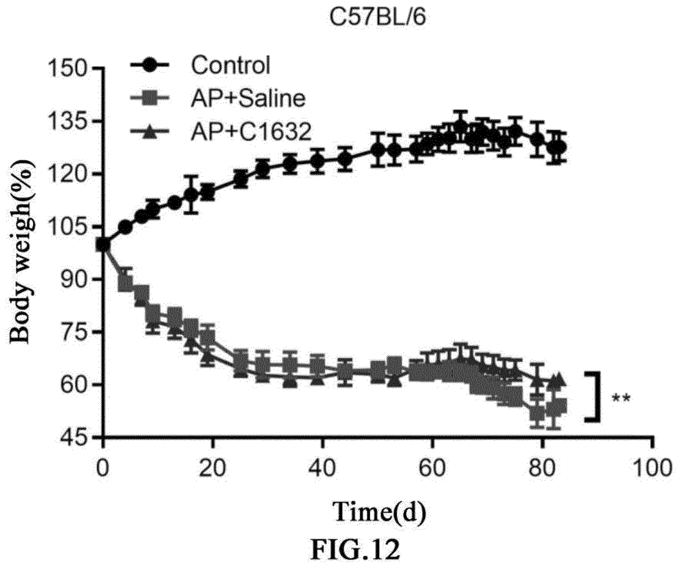

4.1
The mice were divided into three groups: ① normal group; (2)AP+Saline group; ③AP+C1632 group, 10 mice in each group. The CKD mice in group ③ were fed with a feed containing 2‰ AP for 1 month and then treated with C1632. C1632 was administered twice a week at a dose of 68mg/kg of body weight each time. As can be seen, C1632 treatment significantly inhibits weight loss in CKD mice compared with control mice (FIG.12).

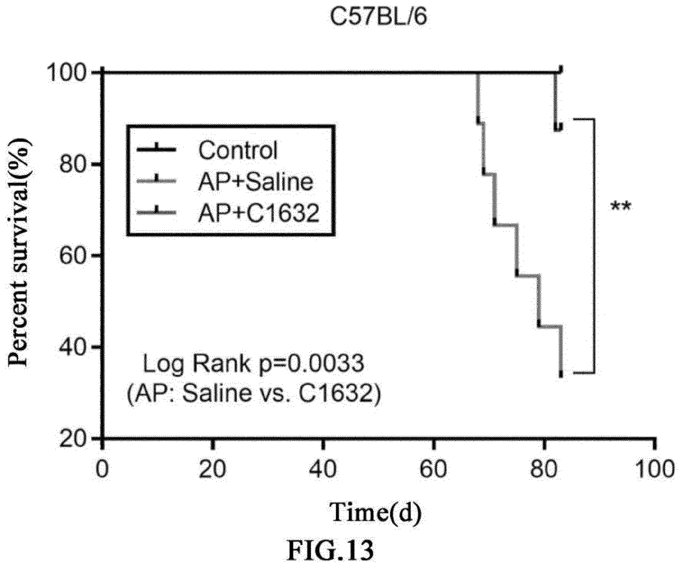

4.2
The mice were divided into three groups: ① normal group; ②AP+saline group; ③AP+C1632 group, 10 mice in each group. The CKD mice in group ② and group ③ were fed with a feed containing 2‰ AP for 1 month and then treated with C1632. C1632 was administered twice a week at a dose of 68mg/kg of body weight each time. During the subsequent 83 days of treatment, the survival of the mice was counted. The results show that C1632 significantly reduces the mortality of the CKD model mice (FIG. 13).

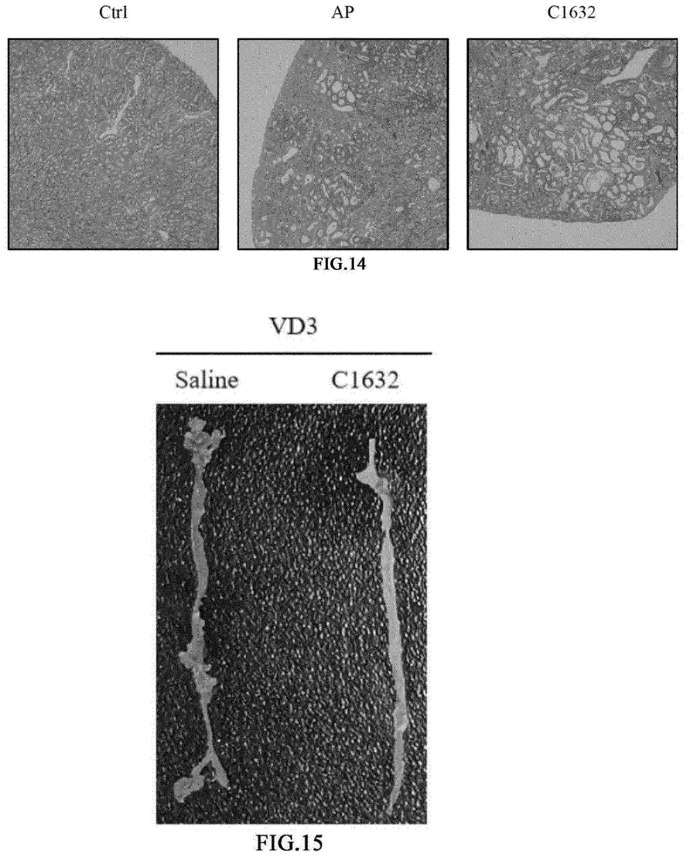

4.3
The mice were divided into three groups: ① normal group; ②AP+saline group; ③AP+C1632 group, 10 mice in each group. The CKD mice in group ② and group ③ were fed with a feed containing 2‰ AP for 1 month and then treated with C1632. C1632 was administered twice a week at a dose of 68mg/kg of body weight each time. After 3 months, the mice were sacrificed, and the kidneys were removed for Masson staining. The results show that the kidneys of control mice mainly show red muscle fibers, while the renal tissue of the saline-treated CKD model mice contains a large number of blue collagen fibers; and C1632 treatment significantly inhibits the level of collagen fibers in the kidney and inhibits the progression of kidney fibrosis in mice (FIG. 14).

### Example 5 Therapeutic effect of C1632 on vascular calcification

C57BL/c mice were intraperitoneally injected with VD3 at a dose of 500 IU/g/ day for 2 consecutive days, and then divided into: ①Saline group; ③C1632 group, 10 mice in each group. C1632 was administered twice a week, and CKD mice were treated with C1632 at a dose of 68mg/kg of body weight each time. After 6 days, the mice were sacrificed, and the aortic blood vessels were dissected for alizarin red staining and hydroxyapatite staining. The results show that C1632 treatment significantly inhibits the progression of aortic vascular calcification (FIG. 15).

The above specific embodiments describe the present disclosure in detail, but the present disclosure is not limited to the above embodiments. Various changes can be made within the knowledge scope of those of ordinary skill in the art without departing from the gist of the present disclosure. In addition, the embodiments of the present disclosure and the features in the embodiments may be combined with each other without conflict.

## Claims

1. A [1,2,4] triazolo [4,3-B] pyridazine derivative for use in the prevention and/or treatment of senescence and/or senescence-related diseases, wherein the [1,2,4] triazolo [4,3-B] pyridazine derivative is a compound of formula (I) or a pharmaceutically acceptable salt thereof: 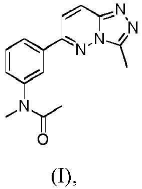 wherein the senescence-related diseases comprise at least one of atherosclerosis, vascular calcification, diabetes, autoimmune diseases, osteoarthritis, Alzheimer's disease, or tissue and organ fibrosis.

2. The [1,2,4] triazolo [4,3-B] pyridazine derivative for use according to claim 1, wherein the senescence comprises at least one of senility, or senescence in tissues and organs comprising skin, brain, lung, heart, bone marrow, liver and kidney.

3. The [1,2,4] triazolo [4,3-B] pyridazine derivative for use according to claim 1, wherein the [1,2,4] triazolo [4,3-B] pyridazine derivative inhibits cellular senescence.

4. The [1,2,4] triazolo [4,3-B] pyridazine derivative for use according to claim 1, wherein the [1,2,4] triazolo [4,3-B] pyridazine derivative relieves an immunosuppressive microenvironment of senescent cells and/or promotes an immunologic clearance of senescent cells.

5. The [1,2,4] triazolo [4,3-B] pyridazine derivative for use according to claim 1, wherein the vascular calcification is aortic vascular calcification.

6. The [1,2,4] triazolo [4,3-B] pyridazine derivative for use according to claim 1, wherein the tissue and organ fibrosis comprises liver fibrosis, lung fibrosis, and kidney fibrosis.

7. The [1,2,4] triazolo [4,3-B] pyridazine derivative for use according to claim 6, wherein the lung fibrosis is idiopathic pulmonary fibrosis.

8. The [1,2,4] triazolo [4,3-B] pyridazine derivative for use according to claim 7, wherein the [1,2,4] triazolo [4,3-B] pyridazine derivative relieves alveolar reduction and reduces an elevated lung density during idiopathic pulmonary fibrosis.

9. The [1,2,4] triazolo [4,3-B] pyridazine derivative for use according to claim 7, wherein the [1,2,4] triazolo [4,3-B] pyridazine derivative inhibits fibroblast proliferation and/or inhibits pulmonary hydroxyproline and/or inhibits immune cell infiltration.

10. The [1,2,4] triazolo [4,3-B] pyridazine derivative for use according to claim 9, wherein the immune cell comprises a CD45-positive cell, a CD68-positive cell, and a CD3-positive cell.

11. The [1,2,4] triazolo [4,3-B] pyridazine derivative for use according to claim 6, wherein the kidney fibrosis is the kidney fibrosis caused by chronic kidney disease.

12. The [1,2,4] triazolo [4,3-B] pyridazine derivative for use according to claim 11, wherein the [1,2,4] triazolo [4,3-B] pyridazine derivative inhibits an abnormal expression of collagen fibers in chronic kidney disease.

13. The [1,2,4] triazolo [4,3-B] pyridazine derivative for use according to claim 1, wherein the [1,2,4] triazolo [4,3-B] pyridazine derivative is administered together with at least one of other components suitable for preventing and/or treating senescence and/or senescence-related diseases.

14. The [1,2,4] triazolo [4,3-B] pyridazine derivative for use according to claim 1, wherein an administration route of the [1,2,4] triazolo [4,3-B] pyridazine derivative comprises at least one of intravenous injection, intraperitoneal injection, intramuscular injection, subcutaneous injection, oral administration, sublingual administration, nasal administration, nebulized administration, or transdermal administration.

15. The [1,2,4] triazolo [4,3-B] pyridazine derivative for use according to claim 1, wherein a dosage form of the [1,2,4] triazolo [4,3-B] pyridazine derivative comprises at least one of suspension, granule, capsule, powder, tablet, emulsion, solution, drop pill, injection, oral preparation, suppository, enema, aerosol, patch or drop.
